# FASCICULE DE BREVET EUROPEEN

(11) **EP 2 490 610 B1**
(45) Date de publication et mention de la délivrance du brevet: **25.06.2014**
(21) Numéro de dépôt: 10785147.9
(22) Date de dépôt: 20.10.2010
(51) Int. Cl.: A61B 17/80, A61F 2/42

(54) **MATERIEL PROTHETIQUE POUR REMPLACER AU MOINS UNE PARTIE DE LA GLENE RADIALE D'UN RADIUS**
PROTHESENMATERIAL FÜR DEN ERSATZ VON MINDESTENS EINEM TEIL DER GELENKPFANNE EINER SPEICHE
PROSTHETIC MATERIAL FOR REPLACING AT LEAST ONE PORTION OF THE RADIAL GLENOID OF A RADIUS

(30) Priorité: 21.10.2009 FR 0905043
(43) Date de publication de la demande: 29.08.2012
(73) Titulaire: D.L.P., 44115 Haute Goulaine (FR)
(72) Inventeur: LIVERNEAUX, Philippe, F-67000 Strasbourg (FR); PODGORSKI, Jean-Pierre, F-49230 Saint Crespin Sur Moine (FR); LARCHE, Grégoire, F-49300 Cholet (FR)
(74) Mandataire: Coralis Harle
(86) Numéro de dépôt international: PCT/FR2010/052236
(87) Numéro de publication internationale: WO 2011/048331

(56) Documents cités:
- WO-A2-2007/047230
- WO-A2-2007/106358
- US-A- 5 522 902
- US-A1- 2007 055 381

## Description

La présente invention concerne un matériel prothétique pour le remplacement d'au moins une partie de la glène radiale d'un radius.

Certaines pathologies articulaires de la partie distale d'un radius (lésions de la glène radiale notamment traumatiques et/ou arthrosiques) peuvent être traitées au moyen de prothèses appropriées, mises en place après découpe osseuse et maintenues au moyen d'un tenon axial solidaire de ladite prothèse, implanté dans le matériau osseux de réception (voir notamment les documents US-2007/0055381 et WO-2007/047230).

Très généralement, certain des éléments de ces dispositifs sont destinés à être fixés à l'extrémité du radius, et d'autres éléments sont destinés à être fixés sur les parties osseuses en regard de la main (os du carpe).

Cependant, ces prothèses sacrifient d'une part une partie du stock osseux du radius distal, et d'autre part sacrifient le cartilage de la première rangée du carpe.

Par ailleurs, en cas de fracture, cette partie distale de radius est généralement réparée au moyen de vis ou de broches, associées éventuellement à une plaque rapportée sur la corticale osseuse.

Mais si la fracture s'accompagne de lésions au niveau de la surface articulaire du radius (lésions antérieures ou occasionnées simultanément à la fracture) on ne dispose alors pas de moyens aptes à réparer ou reconstruire efficacement cette surface articulaire.

La présente invention propose un matériel prothétique pour le traitement des surfaces articulaires abimées des parties distales de radius, qui est relativement simple à poser et qui est relativement peu traumatisant pour le patient.

Ce matériel prothétique présente aussi l'intérêt de permettre la réduction de fracture(s) présente(s) au niveau de cette zone osseuse.

Pour cela, le matériel prothétique conforme à l'invention comprend :
- une structure prothétique constituée d'une plaque prolongée par un embout, ladite plaque étant composée d'un corps plan prolongé par une tête monobloc, et ladite plaque comportant une face de dessus et une face de dessous, et étant munie d'orifices traversants, ladite tête de plaque se prolongeant elle-même par ledit embout, et
- un jeu de vis destinées à coopérer avec lesdits orifices traversants de ladite plaque, pour assurer la fixation de ladite structure prothétique sur le matériau osseux de réception, après résection de la partie de glène radiale que l'on souhaite remplacer et positionnement de la face de dessous de ladite plaque contre la corticale dudit matériau osseux ;
et ce matériel est caractérisé par le fait que ledit embout est constitué - d'une extension support réalisée monobloc avec ladite tête de plaque, et dont la face interne s'étend dans un plan général D qui est décalé angulairement par rapport au plan P dudit corps de plaque, et - d'une platine articulaire rapportée, dont la surface externe, qui se situe dans le prolongement de la face de dessus de ladite plaque, reproduit au moins approximativement au moins une partie de la surface de la glène radiale du radius, laquelle extension support et laquelle platine rapportée sont munies de moyens qui permettent leur solidarisation, lesquels moyens de solidarisation comprennent (i) une structure de glissière(s), ménagée pour une partie sur la face arrière et/ou sur les côtés de ladite platine, et pour une autre partie sur la face avant et/ou sur les côtés de ladite extension, et (ii) des moyens pour fixer en position correcte ladite platine rapportée sur ladite extension.

La face interne de l'extension support s'étend avantageusement dans un plan général D qui est décalé d'un angle compris entre 60° et 80° par rapport au plan P du corps de plaque. D'autre part, le plan de translation de la structure de glissière(s) se situe de préférence dans un plan parallèle à ce plan D.

La plaque de la structure prothétique est avantageusement agencée pour venir se positionner contre la corticale antérieure du radius ; la structure de l'embout, avec les glissières de positionnement de la platine rapportée facilite sensiblement la pose du matériel par la praticien, au niveau du champ opératoire qui est très généralement difficile d'accès.

L'extension de plaque comporte avantageusement deux rails latéraux parallèles, orientés en regard l'un de l'autre, aptes à coopérer avec deux nervures parallèles ménagées sur les côtés de ladite platine rapportée.

D'autre part, les moyens pour fixer en position correcte la platine sur l'extension comprennent avantageusement des butées ménagées au niveau de l'une des extrémités des nervures de la platine rapportée. Ces moyens de fixation comprennent encore de préférence des moyens de verrouillage, du type activables/désactivables, interdisant tout mouvement de la platine rapportée, une fois celle-ci en place sur la plaque (ces moyens de verrouillage peuvent consister en une vis venant se loger dans un orifice taraudé ménagé dans la tête de plaque et dont une partie de l'extrémité supérieure vient en butée contre l'extrémité supérieure de la platine rapportée).

Selon un mode de réalisation préféré, certaines aux moins des vis de la tête de plaque ont une longueur adaptée pour atteindre la corticale opposée du radius, de manière à permettre le maintien d'une réduction osseuse.

Selon une forme de réalisation préférée, la largeur de l'embout est inférieure à la largeur de la glène radiale, ledit embout étant agencé pour venir se positionner dans une réservation ménagée dans la partie centrale de ladite glène radiale, entre la styloïde radiale et l'articulation radio-ulnaire.

L'invention sera encore illustrée, sans être aucunement limitée, par la description suivante d'une forme de réalisation particulière, donnée uniquement à titre d'exemple et représentée sur les dessins annexés dans lesquels :
- la figure 1 est une vue en perspective montrant le matériel prothétique selon l'invention, en deux parties ici dissociées (plaque et platine rapportée), et avec une partie des vis de fixation, positionné au-dessus d'un radius convenablement préparé pour sa réception ;
- la figure 2 est une vue en perspective de la platine rapportée, illustrée ici avec sa face arrière visible,
- la figure 3 est une vue en perspective illustrant le matériel prothétique posé sur l'extrémité distale du radius, avec une partie des vis de fixation pré-positionnées,
- la figure 4 est une vue en perspective similaire à la figure 3, avec toutes les vis de fixation mises en place.

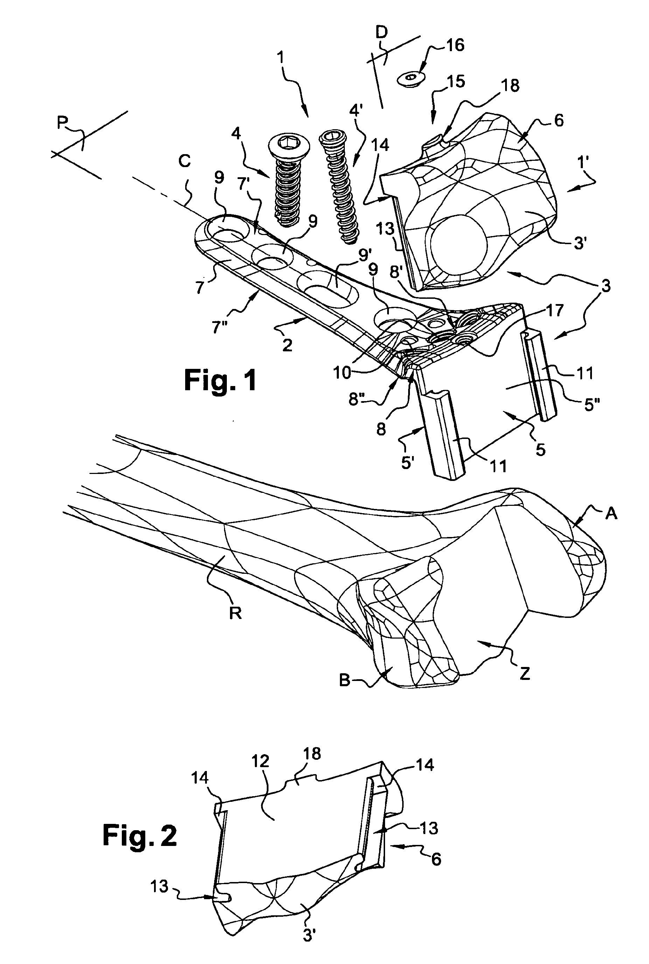

Le matériel prothétique 1, illustré sur les figures 1 à 4, se compose d'une structure prothétique 1' formée - d'une plaque 2 prolongée par - un embout 3 dont la face externe avant 3' reproduit une partie de la surface de la glène radiale d'un radius, cet ensemble étant agencé pour que ladite plaque 2 puisse être fixée, au moyen de vis 4, 4' contre la corticale antérieure d'un radius R, avec ledit embout 3 positionné contre une zone de réception Z correspondant à une portion osseuse dégradée, réséquée par un chirurgien, de sorte à remplacer cette portion osseuse.

Comme on peut le voir sur les figures 1, 3 et 4, la zone de réception Z peut correspondre à une réservation ménagée dans la partie centrale de la glène radiale, entre la styloïde radiale A et l'articulation radio-ulnaire B.

La structure prothétique 1' est en fait ici composée de deux éléments distincts, le premier formé de la plaque 2, munie d'une extension 5 constituant une partie de l'embout 3, et le second formé d'une platine 6 constituant l'autre partie dudit embout 3.

Comme décrit plus loin cette extension 5 et cette platine 6 comportent des moyens qui permettent leur solidarisation.

La plaque 2 est composée d'un corps allongé 7, prolongé à l'une de ses extrémités par une tête monobloc 8, elle-même prolongée par l'extension monobloc précitée 5.

Le corps 7 et la tête 8 de la plaque 2 s'étendent pratiquement dans un même plan P. Ils sont délimités par une face de dessus, respectivement 7' et 8', et par une face de dessous 7" et 8". Ces faces de dessous 7", 8" sont adaptées à l'anatomie de l'extrémité du radius (pour cela, la tête 8 remonte légèrement par rapport au plan dans lequel s'étend le corps de plaque 7).

Le corps de plaque 7 est traversé par une pluralité d'orifices 9, 9' alignés sur son axe C. L'un de ces orifices 9' est de forme générale oblongue ; il est de préférence positionné dans la partie centrale du corps de plaque 7. Les autres orifices 9, ménagés de part et d'autre de l'orifice oblong 9', ont une forme générale circulaire.

Les orifices 9, 9' sont destinés à recevoir des vis de fixation 4, dont une seule est représentée sur la figure 1.

La tête de plaque 8 est également traversée par une pluralité d'orifices 10 (en l'occurrence trois, ici alignés sur sa largeur, c'est-à-dire transversalement à l'axe C du corps de plaque 7).

Ces orifices 10 sont destinés à recevoir des vis 4' (dont une seule est représentée sur la figure 1), adaptées en particulier pour le maintien d'une réduction de fracture osseuse.

L'extension 5 s'étend du côté de la face de dessous 7", 8" du corps de plaque 7 et de la tête de plaque 8, à partir de l'extrémité de ladite tête de plaque 8, ceci selon un plan D qui est décalé angulairement par rapport au plan P du corps de plaque 7. Ici, le décalage angulaire correspondant est de l'ordre de 75°.

Cette extension 5 consiste en un plateau dont la face arrière 5' est disposée du côté des faces de dessous 7" et 8" du corps 7 et de la tête 8 de la plaque 2, et dont la face avant 5" prolonge les faces de dessus 7', 8' dudit corps et de ladite tête de plaque 7, 8.

Les faces arrière 5' et avant 5" de l'extension 5 sont parallèles et elles s'étendent toutes les deux dans le plan D.

La face avant 5" de l'extension 5 comporte des moyens qui permettent la réception et la fixation de l'embout 3, ces moyens se présentant sous la forme de deux rails parallèles 11 orientés en regard l'un de l'autre. Ces deux rails 11 s'étendent parallèlement au plan D de l'extension 5, et le long des bordures latérales de cette dernière.

La plaque 2 peut être réalisée en matériau métallique tel que le titane par exemple.

La platine 6, illustrée isolément sur la figure 2, comporte une face avant 3' qui constitue la face avant de l'embout 3 et qui reproduit au moins approximativement la partie de la surface de la glène radiale du radius ; sa face arrière 12 est adaptée pour venir se solidariser avec la face avant 5" de l'extension 5 de la plaque 2.

A cet effet, la face arrière 12 de la platine 6 est plane et elle comporte, sur ses côtés, deux nervures parallèles 13 aptes à venir s'insérer par coulissement dans les rails 11 de l'extension 5.

Les nervures 13 de la platine 6 et les rails 11 de l'extension 5 forment ainsi une structure de glissières qui permet le montage de la platine 6 sur l'extrémité de la plaque 2, selon un axe (ou un plan) ici parallèle au plan D.

Dans la partie supérieure des nervures 13, on remarque la présence de butées 14 qui permettent de bloquer la progression de la platine 6 sur l'extension 5, par contact avec l'extrémité supérieure des rails 11.

La platine 6 est positionnée sur la plaque 2 par le dessus des rails 11 (c'est-à-dire du côté de la face de dessus 7', 8' du corps de plaque 7 et de la tête de plaque 8).

Les butées 14 bloquent donc la progression de la platine 6 vers le bas, si on tient compte de l'orientation des éléments sur la figure 1.

Des moyens anti-retour 15, du type activables/désactivables, sont prévus pour assurer le verrouillage complet de la platine 6, une fois celle-ci correctement positionnée sur la plaque 2.

Ces moyens de verrouillage 15 sont prévus ici au niveau de l'extrémité de la tête de plaque 8 ; ils consistent en une vis 16 apte à venir se loger dans un orifice taraudé 17 ménagé dans la tête de plaque 8, et dont une partie de l'extrémité supérieure (tête de vis) est agencée pour venir en butée contre la bordure supérieure de la platine rapportée 6. En l'occurrence, la vis 16 vient en butée contre un ergot 18 qui s'étend en légère saillie à partir de la bordure supérieure de la platine 6, empêchant ainsi, en combinaison avec les butées 14, toute possibilité de mouvement de ladite platine 6 une fois en position.

Une fois la platine 6 fixée sur l'extension 5, sa face arrière plane 12 est disposée en regard de la face avant plane 5" de l'extension 5, en contact ou pratiquement en contact avec cette dernière.

L'embout 3 ainsi formé comprend une face avant externe 3' reconstituant la surface de glène radiale réséquée, et une face interne 5' correspondant à la face arrière de l'extension 5, qui s'étend dans le plan D.

La platine 6 peut être réalisée en tout matériau biocompatible, par exemple en chrome-cobalt, peek, polyéthylène ou pyrocarbone.

De préférence, en association avec la plaque 2, il est prévu un jeu de platines 6 d'épaisseurs différentes, mis à la disposition du chirurgien, de manière à lui proposer un choix de prothèses, en fonction de la profondeur de résection qu'il est amené à réaliser dans l'os.

Le matériel prothétique selon l'invention trouve une application particulièrement intéressante dans le cas d'une fracture de l'extrémité distale du radius, accompagnée de lésions de la surface articulaire (au niveau de la glène radiale).

Le chirurgien peut alors utiliser avantageusement une technique opératoire par voie palmaire.

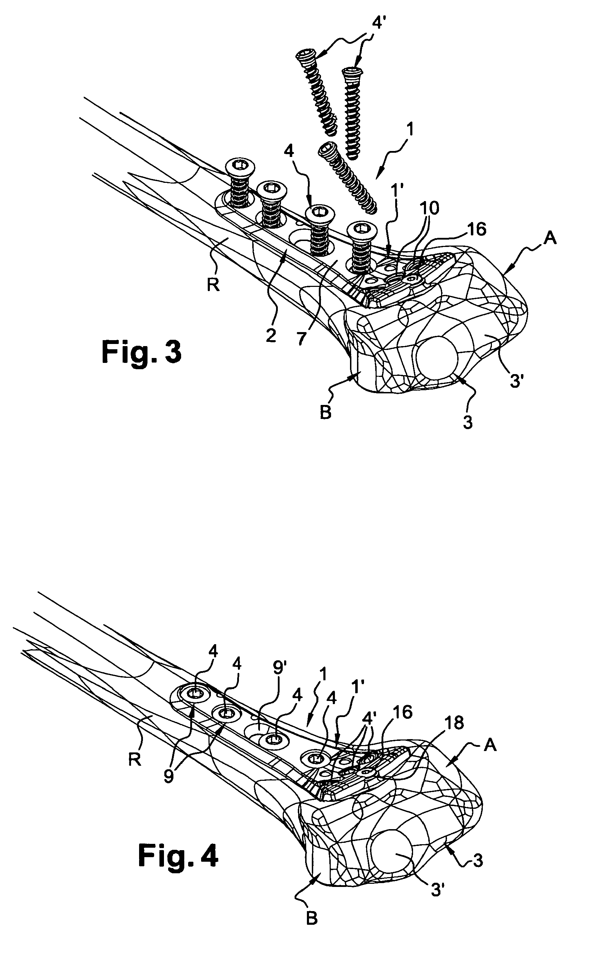

Pour cela, après ouverture au niveau du poignet du patient, le praticien réalise la résection de la glène radiale abimée, ainsi qu'une réduction manuelle de la fracture osseuse. Ensuite, il positionne convenablement la plaque 2 sur la corticale antérieure du radius, avec l'extension 5 et la platine 6 associée, dans la réservation Z (figure 3) Avant de refermer le champ opératoire ;

Plus précisément, dans un premier temps, la plaque 2, sans la platine 6, peut être prépositionnée sur le radius en utilisant simplement une vis centrale 4 logée dans l'orifice oblong 9'. Par suivi sous amplificateur de brillance, cela permet au chirurgien de tester différentes platines 6, d'ajuster la position de la plaque 2 sur le radius et de réduire temporairement la fracture au moyen de broches.

Lorsque le choix du positionnement de la plaque 2 est réalisé, le chirurgien la fixe correctement au moyen des vis 4 dans les orifices 9 du corps de plaque 7 et au moyen des vis 4' dans les orifices 10 de la tête de plaque 8 (figures 3 et 4). Comme indiqué précédemment, ces dernières sont agencées pour assurer le maintien de la réduction de la fracture osseuse. Dans ce cadre, leur longueur est adaptée pour atteindre la corticale opposée du radius ; ces vis 4' peuvent être verrouillées à la plaque 2 par l'intermédiaire d'un système de verrouillage poly-axial.

Le chirurgien peut ensuite terminer l'opération en effectuant la balance ligamentaire, puis en positionnant la platine 6 définitive sur l'extension de plaque 5, avant de refermer le champ opératoire.

Dans une variante de réalisation, la plaque 2 peut être réalisée en plusieurs parties munies de moyens d'assemblage entre elles.

## Revendications

1. Matériel prothétique pour remplacer au moins une partie de la glène radiale d'un radius (R), lequel matériau comprend :
- une structure prothétique (1') constituée d'une plaque (2) prolongée par un embout (3), ladite plaque (2) étant composée d'un corps plan (7) prolongé par une tête monobloc (8), et ladite plaque (2) comportant une face de dessus (7', 8') et une face de dessous (7", 8"), et étant munie d'orifices traversants (9, 9', 10), ladite tête de plaque (8) se prolongeant elle-même par ledit embout (3), et
- un jeu de vis (4, 4') destinées à coopérer avec lesdits orifices traversants (9, 9', 10) de ladite plaque (2), pour assurer la fixation de ladite structure prothétique (1') sur le matériau osseux de réception (R), après résection de la partie de glène radiale que l'on souhaite remplacer et positionnement de la face de dessous (7', 8') de ladite plaque (2) contre la corticale dudit matériau osseux (R),
ledit embout (3) étant constitué - d'une extension support (5) réalisée monobloc avec ladite tête de plaque (8) et dont la face interne (5') s'étend dans un plan général (D) qui est décalé angulairement par rapport au plan (P) dudit corps de plaque (7), et - d'une platine articulaire rapportée (6), dont la surface externe (3'), qui se situe dans le prolongement de la face de dessus (7', 8') de ladite plaque (2), reproduit au moins approximativement au moins une partie de la surface de la glène radiale du radius, laquelle extension support (5) et laquelle platine rapportée (6) sont munies de moyens (11, 13, 14, 15) qui permettent leur solidarisation, lesquels moyens de solidarisation comprennent des moyens (14, 15) pour fixer en position correcte ladite platine rapportée (6) sur ladite extension (5),
**caractérisé en ce que** lesdits moyens de solidarisation comprennent en plus une structure de glissière(s) (11, 13), ménagée pour une partie sur la face arrière et/ou sur les côtés de ladite platine (6), et pour une autre partie sur la face avant (5") et/ou sur les côtés de ladite extension (5).

2. Matériel selon la revendication 1, **caractérisé en ce que** la face interne (5') de l'extension support (5) s'étend dans un plan général (D) décalé d'un angle compris entre 60° et 80° par rapport au plan (P) du corps de plaque (7).

3. Matériel selon l'une quelconque des revendications 1 ou 2, **caractérisé en ce que** le plan de translation de la structure de glissière(s) (11, 13) se situe dans un plan parallèle audit plan (D).

4. Matériel selon l'une quelconque des revendications 1 à 3, **caractérisé en ce que** l'extension de plaque (5) comporte deux rails latéraux parallèles (11), orientés en regard l'un de l'autre, aptes à coopérer avec deux nervures parallèles (13) ménagées sur les côtés de ladite platine rapportée (6).

5. Matériel selon la revendication 4, **caractérisé en ce que** lesdits moyens pour fixer en position correcte la platine (6) sur l'extension (5) comprennent des butées (14) ménagées au niveau de l'une des extrémités des nervures (13) de ladite platine rapportée (6).

6. Matériel selon l'une quelconque des revendications 4 ou 5, **caractérisé en ce que** lesdits moyens pour fixer en position correcte la platine (6) sur l'extension (5) comprennent des moyens de verrouillage (15), du type activables/désactivables, interdisant tout mouvement de la platine rapportée (6) une fois celle-ci en place sur la plaque (2).

7. Matériel selon la revendication 6, **caractérisé en ce que** lesdits moyens de verrouillage (15) consistent en une vis (16) venant se loger dans un orifice taraudé (17) ménagé dans la tête de plaque (8) et dont une partie de l'extrémité supérieure vient en butée contre l'extrémité supérieure de la platine rapportée (6).

8. Matériel selon l'une quelconque des revendications 1 à 7, **caractérisé en ce que** la plaque (2) de la structure prothétique (1') est agencée pour venir se positionner contre la corticale antérieure du radius (R).

9. Matériel selon l'une quelconque des revendications 1à 8, **caractérisé en ce que** certaines au moins des vis (4') de la tête de plaque (8) ont une longueur adaptée pour atteindre la corticale opposée du radius (R), de manière à permettre le maintien d'une réduction osseuse.

10. Matériel selon l'une quelconque des revendications 1 à 9, **caractérisé en ce que** la largeur de l'embout (3) est inférieure à la largeur de la glène radiale et **en ce que** ledit embout (3) est agencé pour venir se positionner dans une réservation (Z) ménagée dans la partie centrale de ladite glène radiale, entre la styloïde radiale et l'articulation radio-ulnaire.

## Patentansprüche

1. Prothesenmaterial für den Ersatz von mindestens einem Teil der Gelenkpfanne einer Speiche (R), wobei das Material Folgendes umfasst:
- einen Prothesenaufbau (1'), der aus einer Platte (2) besteht, die durch ein Ansatzstück (3) verlängert wird, wobei die Platte (2) aus einem ebenen Körper (7) besteht, der durch einen einstückigen Kopf (8) verlängert wird, und wobei die Platte (2) eine Oberseite (7', 8') und eine Unterseite (7", 8") umfasst und mit Durchbrechungen (9, 9', 10) versehen ist, wobei der Plattenkopf (8) seinerseits durch das Ansatzstück (3) verlängert wird, und
- einen Satz (4, 4') von Schrauben, die dazu gedacht sind, mit den Durchbrechungen (9, 9', 10) der Platte (2) zusammenzuwirken, um die Befestigung des Prothesenaufbaus (1') an dem Aufnahmeknochenmaterial (R) nach einer Resektion des Gelenkpfannenteils, den man ersetzen möchte, und der Positionierung der Unterseite (7', 8') der Platte (2) gegen die Kortikalis des Knochenmaterials (R) sicherzustellen,
wobei das Ansatzstück (3) - aus einer Trägerverlängerung (5), die einstückig mit dem Plattenkopf (8) ausgebildet ist und deren interne Seite (5') sich in einer Hauptebene (D) erstreckt, die im Verhältnis zur Ebene (P) des Plattenkörpers (7) winkelmäßig versetzt ist, und - aus einer angesetzten Gelenkplatine (6), deren externe Oberfläche (3'), die sich in der Verlängerung der Oberseite (7', 8') der Platte (2) befindet, mindestens annähernd mindestens einen Teil der Oberfläche der Gelenkpfanne der Speiche wiedergibt, besteht, wobei die Trägerverlängerung (5) und die angesetzte Platine (6) mit Mitteln (11, 13, 14, 15) versehen sind, die ihre feste Verbindung ermöglichen, wobei die Mittel zum festen Verbinden Mittel (14, 15) umfassen, um die angesetzte Platine (6) auf der Verlängerung (5) in der richtigen Position zu fixieren,
**dadurch gekennzeichnet, dass** die Mittel zum festen Verbinden ferner einen Aufbau (11, 13) aus einer oder mehreren Gleitschienen umfassen, der zum Einen auf der Rückseite und/oder auf den Seiten der Platine (6) und zum Anderen auf der Vorderseite (5") und/oder auf den Seiten der Verlängerung (5) eingerichtet ist.

2. Material nach Anspruch 1, **dadurch gekennzeichnet, dass** sich die interne Seite (5') der Trägerverlängerung (5) in einer Hauptebene (D) erstreckt, die um einen Winkel zwischen 60° und 80° im Verhältnis zu der Ebene (P) des Plattenkörpers (7) versetzt ist.

3. Material nach einem der Ansprüche 1 oder 2, **dadurch gekennzeichnet, dass** sich die Translationsebene des Gleitschienenaufbaus (11, 13) in einer Ebene parallel zu der Ebene (D) befindet.

4. Material nach einem der Ansprüche 1 bis 3, **dadurch gekennzeichnet, dass** die Plattenverlängerung (5) zwei parallele seitliche Schienen (11) umfasst, die einander gegenüberliegend orientiert sind und in der Lage sind, mit zwei parallelen Rippen (13) zusammenzuwirken, die auf den Seiten der angesetzten Platine (6) eingerichtet sind.

5. Material nach Anspruch 4, **dadurch gekennzeichnet, dass** die Mittel zum Befestigen der Platine (6) in der richtigen Position auf der Verlängerung (5) Anschläge (14) umfassen, die an einem der Enden der Rippen (13) der angesetzten Platine (6) eingerichtet sind.

6. Material nach einem der Ansprüche 4 oder 5, **dadurch gekennzeichnet, dass** die Mittel zum Befestigen der Platine (6) in der richtigen Position auf der Verlängerung (5) Verriegelungsmittel (15) aktivierbarer / deaktivierbarer Art umfassen, die jegliche Bewegung der angesetzten Platine (6) untersagen, sobald diese auf der Platte (2) liegt.

7. Material nach Anspruch 6, **dadurch gekennzeichnet, dass** die Verriegelungsmittel (15) aus einer Schraube (16) bestehen, die in eine Gewindeöffnung (17) passt, die in dem Plattenkopf (8) eingerichtet ist und von der ein Teil des oberen Endes an dem oberen Ende der angesetzten Platine (6) anschlägt.

8. Material nach einem der Ansprüche 1 bis 7, **dadurch gekennzeichnet, dass** die Platte (2) des Prothesenaufbaus (1') angeordnet ist, um sich an der vorderen Kortikalis der Speiche (R) zu positionieren.

9. Material nach einem der Ansprüche 1 bis 8, **dadurch gekennzeichnet, dass** mindestens einige der Schrauben (4') des Plattenkopfs (8) eine Länge aufweisen, die geeignet ist, um die gegenüberliegende Kortikalis der Speiche (R) zu erreichen, um den Erhalt einer Knochenreduktion zu ermöglichen.

10. Material nach einem der Ansprüche 1 bis 9, **dadurch gekennzeichnet, dass** die Breite des Ansatzstücks (3) kleiner als die Breite der Gelenkpfanne ist, und dass das Ansatzstück (3) angeordnet ist, um sich in einer Aussparung (Z) zu positionieren, die in dem zentralen Teil der Gelenkpfanne zwischen dem Griffelfortsatz und dem Radioulnargelenk eingerichtet ist.

## Claims

1. Prosthetic material for replacing at least one portion of the radial glenoid of a radius (R), which material comprises:
- a prosthetic structure (1') constituted by a plate (2) extended by an endpiece (3), said plate (2) being made up of a planar body (7) extended by a one-piece head (8), and said plate (2) including a top face (7', 8') and a bottom face (7", 8"), and being provided with through orifices (9, 9', 10), said plate head (8) itself being extended by said endpiece (3), and
- a set of screws (4, 4') for co-operating with said through orifices (9, 9', 10) of said plate (2) to fasten said prosthetic structure (1') to the receiving bony material (R) after resection of the portion of the radial glenoid that it is desired to replace and after positioning the bottom face (7", 8") of said plate (2) against the cortex of said bone material (R),
said endpiece (3) being constituted by - a support extension (5) made as one piece with said plate head (8) and having an inner face (5') extending in a general plane (D) that is offset angularly relative to the plane (P) of said plate body (7), and by - a fitted articular panel (6) having an outer surface (3') that is situated extending the top face (7', 8') of said plate (2) and reproducing at least approximately at least a portion of the surface of the radial glenoid of the radius, which support extension (5) and fitted panel (6) are provided with means (11, 13, 14, 15) enabling them to be secured to each other, which securing means comprise means (14, 15) for fastening said fitted panel (6) in the correct position on said extension (5),
the material being **characterized in that** said securing means comprise too a slideway structure (11, 13) arranged in part on the rear face and/or on the sides of said panel (6), and also in part on the front face (5") and/or on the sides of said extension (5).

2. Material according to claim 1, **characterized in that** the inner face (5') of the support extension (5) extends in a general plane D that is offset at an angle lying in the range 60° to 80° relative to the plane P of the plate body (7).

3. Material according to any one of claims 1 or 2, **characterized in that** the plane of movement in translation of the slideway structure (11, 13) lies in a plane parallel to said plane (D).

4. Material according to any one of claims 1 to 3, **characterized in that** the plate extension (5) has two parallel side rails (11) facing each other and suitable for co-operating with two parallel ribs (13) arranged on the sides of said fitted panel (6).

5. Material according to claim 4, **characterized in that** said means for fastening the panel (6) in the correct position on the extension (5) comprise abutments (14) formed at one of the ends of the ribs (13) of said fitted panel (6).

6. Material according to any one of claims 4 or 5, **characterized in that** said means for fastening the panel (6) in the correct position on the extension (5) comprise locking means (15) of the activatable/deactivatable type that prevent any movement of the fitted panel (6) once it is in place on the plate (2).

7. Material according to claim 6, **characterized in that** said locking means (15) consist of a screw (16) that is received in a tapped orifice (17) formed in the plate head (8) and having a top end portion that comes into abutment against the top end of the fitted panel (6).

8. Material according to any one of claims 1 to 7, **characterized in that** the plate (2) of the prosthetic structure (1') is arranged to be positioned against the anterior cortex of the radius (R).

9. Material according to any one of claims 1 to 8, **characterized in that** at least some of the screws (4') of the plate head (8) are of a length that is adapted for reaching the opposite cortex of the radius (R) so as to enable a bone reduction to be held.

10. Material according to any one of claims 1 to 9, **characterized in that** the width of the endpiece (3) is less than the width of the radial glenoid, and **in that** said endpiece (3) is arranged to be positioned in a reception zone (Z) arranged in the central portion of said radial glenoid, between the radial styloid and the radioulnar joint.
